# EUROPEAN PATENT APPLICATION

(11) **EP 4 289 444 A1**
(43) Date of publication of application: **13.12.2023**
(21) Application number: 21924566.9
(22) Date of filing: 02.02.2021
(51) Int. Cl.: A61K 45/06, A61K 31/122, A61K 31/353, A61K 31/355, A61K 31/375, A61K 31/426, A61K 31/444, A61K 31/519, A61P 31/12

(54) **ANTI-VIRAL AGENT**

(71) Applicant: Nesa LLC, Tokyo, 113-0031 (JP); Nezu Life Science Co., Ltd., Toshima-ku Tokyo 171-0033 (JP)
(72) Inventor: NISHINO, Takeshi, Tokyo 113-0021 (JP); KUSANO, Teruo, Tokyo 113-8602 (JP)
(74) Representative: Wächtershäuser & Hartz Patentanwaltspartnerschaft mbB
(86) International application number: PCT/JP2021/003756
(87) International publication number: WO 2022/168169

(57) **Abstract**

To provide a novel antiviral agent. The antiviral agent contains a xanthine oxidase inhibitor in combination with an active oxygen scavenger.

## Description

### Technical Field

The present invention relates to a novel antiviral agent.

### Background Art

Vaccination is a basic approach to most viral infectious diseases, although some viral infectious diseases, such as influenza infection, are treated with therapeutic agents. However, a large amount of time and costs are involved in production of such vaccines, and vaccination may evoke side effects such as anaphylactic shock. Furthermore, vaccination is impotent to some patients. Thus, generally, difficulty is encountered in preventing viral infection, and a therapeutic method therefor is limited (see Non-Patent Document 1).

### Citation List

### Non-Patent Documents

Non-Patent Document 1: Web site of The Japan Association for Infectious Diseases
Non-Patent Document 2: J. Biol. Chem. 265, 14170-14175 (1990)
Non-Patent Document 3: British Journal of Nutrition, 84(S1), S19-S25 (2000)
Non-Patent Document 4: ACS Omega 5,16292-16298 (2020)
Non-Patent Document 5: J. Cell Biol. 118, 227-244 (1992)

### Summary of the Invention

### Problems to be Solved by the Invention

An object of the present invention is to provide an antiviral agent acting based on a completely new mechanism.

### Means for Solving the Problems

The present inventors have studied by focusing on a protein site which is essentially involved in viral infection and which is included in a virus or in a receptor present at a site of infection (hereinafter referred to as an "infection site") of a human body. As a result, the inventors have found that use of a xanthine oxidase inhibitor and an active oxygen scavenger in combination is effective in the case where the receptor present at an infection site of a human body includes a protein site which is essentially involved in viral infection and which has a disulfide bond (S-S bond), or is effective for a virus having a disulfide bond (S-S bond) at a protein site which is essentially involved in viral infection and which is included in the virus. Also, the above combined use is effective for a viral infectious disease in which an S-S bond plays a role in a part of such receptors. The present invention has been accomplished on the basis of these findings.

Accordingly, the present invention provides the following inventions [1] to [8].
[1] An antiviral agent containing a xanthine oxidase inhibitor in combination with an active oxygen scavenger.
[2] The antiviral agent as described in [1] above, wherein the xanthine oxidase inhibitor is one or more members selected from the group consisting of Allopurinol, Febuxostat, and Topiroxostat.
[3] The antiviral agent as described in [1] or [2] above, wherein the active oxygen scavenger is one or more members selected from the group consisting of a reduced-form thiol agent, ascorbic acid or a derivative thereof, a tocopherol, an astaxanthine, and a flavonoid.
[4] The antiviral agent as described in any one of [1] to [3] above, which is an antiviral agent composition containing a xanthine oxidase inhibitor and an active oxygen scavenger.
[5] The antiviral agent as described in any one of [1] to [3] above, which is a combination of a composition containing a xanthine oxidase inhibitor with a composition containing an active oxygen scavenger.
[6] Use, for producing an antiviral agent, of a xanthine oxidase inhibitor in combination with an active oxygen scavenger.
[7] A combination, for use in prevention or treatment of a viral infectious disease, of a xanthine oxidase inhibitor with an active oxygen scavenger.
[8] A method for preventing or treating a viral infectious disease, comprising administering to a subject in need thereof a xanthine oxidase inhibitor in combination with an active oxygen scavenger.

### Effects of the Invention

The antiviral agent of the present invention is effective in the case where a viral receptor present at an infection site of a human body includes a protein site which is essentially involved in viral infection and which has an S-S bond, or is effective for a virus having an S-S bond at a protein site which is essentially involved in viral infection and which is included in the virus. Also, the antiviral agent of the present invention is effective for a viral infectious disease in which an S-S bond plays a role in a part of such a receptor.

### Brief Description of the Drawings

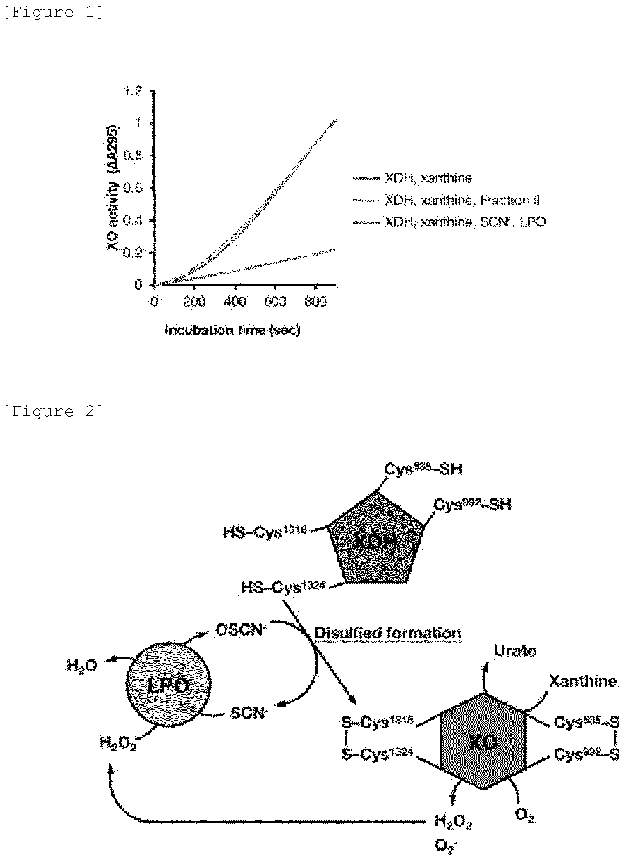

[Fig. 1] A graph showing conversion of xanthine dehydrogenase (XDH) to xanthine oxidase (XO) by the mediation of XDH, xanthine, whey fraction (Fraction II), ⁻SCN, and LPO.
[Fig. 2] A schematic diagram showing the conversion reaction of Fig. 1. The diagram shows that four components: two proteins (i.e., xanthine dehydrogenase (XDH) and lactoperoxidase (LPO)), and thiocyanate and a substrate for xanthine dehydrogenase supplied from blood (i.e., xanthine or hypoxanthine) can convert XDH to XO.

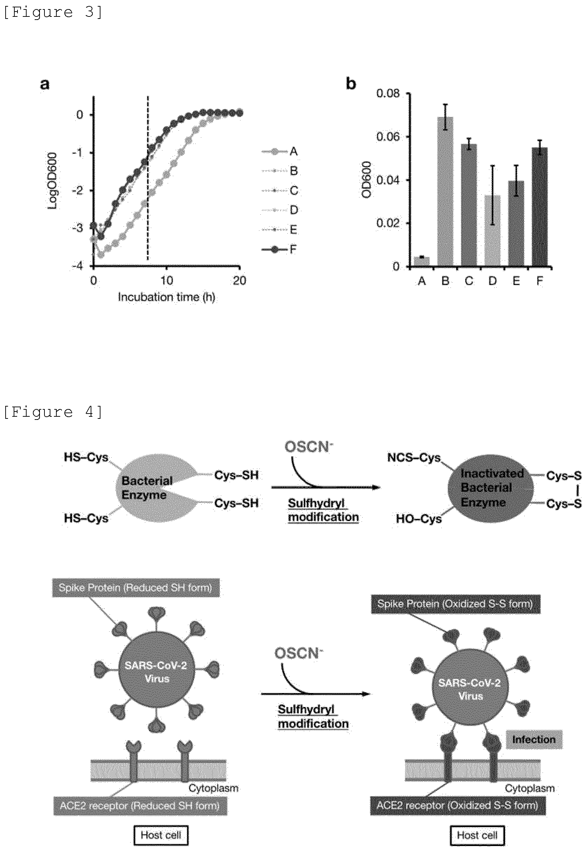

[Fig. 3] Graphs show that the four components: two proteins (i.e., xanthine dehydrogenase (XDH) and lactoperoxidase (LPO)), and thiocyanate and a substrate for xanthine dehydrogenase supplied from blood (i.e., xanthine or hypoxanthine) can convert XDH to XO, and an inhibitory action with respect to growth of bacteria. As is clear from graph "a" of Fig. 3, growth of bacteria is retarded in the presence of all four of the components. Graph "b" of Fig. 3 shows the results of turbidity observation (at OD of 600) under incubation in the case of the broken line in graph "a." In
Fig. 3, A represents a system in the presence of all four of the components; B a system lacking an XDH-type enzyme; C a system lacking a xanthine substrate; D a system lacking NaSCN; E a system lacking LPO; and F a system formed only of a bacterial medium. All the test cases have proven conversion of XDH to XO and the resulting inhibition of bacterial proliferation by a synergic effect of the four components.
[Fig. 4] Fig. 4 shows an anticipated action of ⁻SCN on bacteria (i.e., an action of forming protein S-S bonds in the target bacteria so as to inhibit proliferation thereof) (see upper scheme) and an action of forming S-S bonds in a spike protein of the target virus, to thereby maintain the steric structure thereof (see lower scheme).

### Modes for Carrying Out the Invention

The antiviral agent of the present invention contains, as active ingredients, a xanthine oxidase inhibitor and an active oxygen scavenger in combination.

The xanthine oxidase employed in the present invention includes xanthine oxidase and xanthine dehydrogenase. Examples of the xanthine oxidase inhibitor include one or more species selected from the group consisting of Allopurinol, Febuxostat, and Topiroxostat. Of these, Febuxostat and Topiroxostat are more preferred.

Examples of the active oxygen scavenger include one or more species selected from the group consisting of a reduced-form thiol agent, ascorbic acid or a derivative thereof, a tocopherol, an astaxanthine, and a flavonoid.

Specific examples of the reduced-form thiol agent include reduced-form glutathione, reduced-form cysteine, and Co-Q.

Specific examples of the ascorbic acid or a derivative thereof include ascorbic acid, ascorbic acid fatty acid esters, and ascorbic acid glucosides. Specific examples of the tocopherol include tocopherol, tocopherol acetate, and tocopherol nicotinate. Specific examples of the flavonoid include polyphenols of plant origin such as catechins, anthocyanins, and sesamin.

The antiviral agent of the present invention is effective in the case where a receptor present at an infection site of a human body includes a protein site which is essentially involved in viral infection and which has a disulfide bond (S-S bond), or is effective for a virus having a disulfide bond (S-S bond) at a protein site which is essentially involved in viral infection and which is included in the virus.

Generally, xanthine dehydrogenase (XDH) is present in cytoplasm of cells of the mammary gland, the liver, the kidneys, and other organs. Except for the case of the mammary gland, an extract carefully obtained from the target cells is known to contain xanthine dehydrogenase (XDH) (see Non-Patent Document 2). In the mammary gland, four components are possibly present: originally present dehydrogenase, originally present lactoperoxidase, xanthine or hypoxanthine supplied from blood, and thiocyanate ion which is usually present in the live body and derived from natural products. When the four components are co-present, xanthine dehydrogenase (XDH) is auto-catalytically converted to xanthine oxidase (XO) (see Fig. 1), whereby oxothiocyanate (⁻OSCN) is generated. As has already been known, generally, oxothiocyanate can inhibit proliferation of bacteria, to thereby prevent infection (see Non-Patent Document 3).

In the analysis of the mechanism of preventing bacterial infection, the present inventors previously conducted experiments and found that the following four components: two proteins (i.e., xanthine dehydrogenase and lactoperoxidase), thiocyanate supplied from blood, and a substrate targeted by xanthine dehydrogenase (hypoxanthine or xanthine), are essential to induce transformation of XDH to XO. The inventors searched cells in which the above two proteins (xanthine dehydrogenase and lactoperoxidase) are cogenerated through gene expression, and found that the proteins are present at high density in the salivary gland, respiratory tract mucosa, pulmonary alveolar epithelial cells, and intestinal epithelial cells, and that oxothiocyanate plays a role in preventing bacterial infection in such cells. A conceivable prevention mechanism by oxothiocyanate includes fixation of S-S bonds to key protein molecules present in the bacterial cells, to thereby prevent bacterial proliferation. Also, the inventors investigated the conformation of the spike protein of SARS-CoV-2, which is a COVID-19 causal virus. As a result, a large number of S-S bonds (one primary domain having 12 to 14 S-S bonds: 24 to 28 cysteine residues) were found to sustain stability of the steric structure. Meanwhile, an ACE2 receptor has S-S bonds, which conceivably enhance affinity to spike protein (see Non-Patent Document 4). In an influenza virus, the glycoprotein essential to viral infection is known to have S-S bonds, which play an important role (see Non-Patent Document 5). That is, S-S bonds are essential to infection and copy proliferation of the virus. Since the aforementioned oxothiocyanate can oxidize two or more cysteine SH groups of the viral protein (i.e., oxothiocyanate source), such viruses can conceivably proliferate. Under such circumstances, the present inventors have found that intercepting the supply of the four components results in inhibition of conversion of XDH to XO and 2-step enzymatic reactions (from hypoxanthine to xanthine oxidase, and from xanthine to uric acid). The inventors have further found that administration of reduced-form thiol can inhibit conversion of XDH to XO and enzymatic activity in the 2-step reaction, in the case of COVID-19, and the amount of oxothiocyanate (an intermediate) can be reduced by the co-presence of reduced-form thiol, in the case of influenza viruses. As a result, the inventors have conceived the above techniques. Therefore, administration of a xanthine oxidase inhibitor in combination with an active oxygen scavenger (e.g., a reduced-form thiol agent) is effective for the prevention of viral infection and the treatment of such a viral infectious disease. Particularly, in order to prevent viral infection and treat such a viral infectious disease, washing of the oral cavity or the trachea directly with the agent of the invention, or spraying the agent directly thereto is considered to be effective and, in the case of a viral infectious disease concomitant with gastrointestinal symptoms, peroral administration of the agent is considered to be effective.

The antiviral agent of the present invention is a pharmaceutical agent containing the xanthine oxidase inhibitor in combination with the active oxygen scavenger. No particular limitation is imposed on the form of the agent, so long as these ingredients can be employed in combination. Specifically, on the basis of dosage or regimen of the agent, individual ingredients may be formulated into separate dosage forms or a single dosage form (i.e., a combination drug). Alternatively, in production and sales cases of the agent, individual formulations may be combined in a single package which is suited for combined administration, or may be provided as separate packages. When individual formulations are combined in a single package or provided as separate packages, the products may be provided as kit formulations each including a user's instruction which specifies that the xanthine oxidase inhibitor and the active oxygen scavenger are to be administered in combination. As used herein, the "user's instruction" must specify the doses. Specific examples include a package insert and a pamphlet. In the case of a kit formulation including a user's instruction, the user's instruction may be printed on or inserted into a package thereof. Alternatively, both the antiviral agent of the present invention and a user's instruction may be inserted into a package thereof.

In the case where individual ingredients are formulated as separate dosage forms, a commercially available peroral drug of the xanthine oxidase inhibitor and a commercially available peroral drug of the active oxygen scavenger may be employed as they are. In the case where individual ingredients are combined to form a single dosage form, a pharmaceutical composition containing the xanthine oxidase inhibitor and the active oxygen scavenger (e.g. a formulation containing two ingredients therein as mentioned below) or other dosage forms thereof may be provided.

Also, in a preferred embodiment of the antiviral agent of the present invention, the agent is administered to the oral cavity or the trachea, corresponding to virus infection sites. Specifically, an embodiment which allows the agent to be sprayed or applied to the oral cavity or the trachea and an embodiment which allows the oral cavity or the trachea to be washed with the agent are preferred, with the liquid-form agent being more preferred.

In the antiviral agent of the present invention, the ratio in dose of the xanthine oxidase inhibitor to the active oxygen scavenger, which varies depending on the specific composition of the ingredients, is preferably 1:500 to 500:1, more preferably 1:100 to 100:1, on a mass basis. The daily dose of the xanthine oxidase inhibitor and that of the active oxygen scavenger, which vary depending on the specific composition of the ingredients, are preferably 1 mg to 1 g and 1 mg to 5 g, respectively.

The virus targeted by the antiviral agent of the present invention is preferably a virus where the receptor thereto present at an infection site of a human body includes a protein site which is essentially involved in viral infection and which has a disulfide bond (S-S bond), or a virus having a disulfide bond (S-S bond) at a protein site which is essentially involved in viral infection and which is included in the virus. Specifically, corona viruses, influenza viruses, retroviruses (e.g., HIV), etc. are preferred, with SARS-CoV-2 being particularly preferred.

### Examples

The present invention will next be described in detail by way of examples, which should not be construed as limiting the present invention thereto.

### Example 1

XDH catalyzes the following reaction.
Xanthine + NAD⁺ -> Uric acid + NADH
XO catalyzes the following reaction.
Xanthine + O₂ → Uric acid + H₂O₂ (A small amount of O₂⁻is generated, but undergoes disproportionation to H₂O₂.)
Oxothiocyanate is formed, in the presence of LPO, via the following reaction.
SCN + H₂O₂ -> ⁻OSCN
Oxothiocyanate non-enzymatically causes the following reaction at high rate of reaction.
OSCN + 2RSH -> RS-SR + ⁻SCN

In the absence of or in the presence of an insufficient amount of NAD⁺, XDH maintains weak oxidase activity. When - SCN and LPO are additionally present, conversion of XDH to XO is accelerated via linking of intraenzyme S-S bonds (Cys535 and Cys992 primarily, Cys1316 and Cys1324 secondarily).

As XO increases, formation of ⁻OSCN is promoted, to thereby synergistically promote conversion of XDH to XO. Actually, XDH (20 nM in vitro), which exhibits weak XO activity under atmospheric pressure in the presence of oxygen and 150 µM xanthine, is transformed in an accelerated manner to XO in the presence of a whey fraction in milk, or SCN (5µM) + LPO (1 nM), whereby the XO activity is enhanced.

Fig. 1 shows data of changes in XO activity vs. reaction time (course). The system including all four of the components supplied by reaction demonstrates conversion of XDH to XO. In other systems, each lacking any of the four components, the XDH reaction system assumes formation of uric acid by XDH in a proportional manner.

Fig. 2 is a schematic diagram showing sites at which two S-S bonds are formed. These two S-S bonds are denoted by SH groups 535, 992, 1316, and 1324.

### Example 2

In order to investigate whether or not the four components: xanthine dehydrogenase, lactoperoxidase, thiocyanate and a substrate for xanthine dehydrogenase supplied from blood (i.e., xanthine or hypoxanthine) can inhibit proliferation of bacteria, comparative studies of *E*. *Coli* incubation in a normal oxygen atmosphere were carried out.

Specifically, *E. Coli* was incubated in the presence of 1nM LPO, 5µM NaSCN, 20nM XDH, and/or 150µM xanthine. Fig. 3 shows the results.

As shown in Graph "a" of Fig. 3, growth of *E. Coli* was found to be retarded in the presence of all four of the components. Graph "b" of Fig. 3 shows the results of turbidity observation (at OD of 600) under incubation in the case of the broken line in graph "a."

In Fig. 3, A represents a system in the presence of all four of the components; B a system lacking an XDH-type enzyme; C a system lacking a xanthine substrate; D a system lacking NaSCN; E a system lacking LPO; and F a system formed only of a bacterial medium (M9 minimum medium).

### Example 3

Fig. 4 shows an anticipated action of ⁻SCN on bacteria (i.e., an action of forming protein S-S bonds in the target bacteria so as to inhibit proliferation thereof) and an action of forming S-S bonds in a spike protein of the target virus, to thereby maintain the steric structure thereof.

When the receptor to the virus has S-S bonds, the maintained stearic structure of the spike protein achieves enhanced affinity to the receptor (i.e., interaction). Thus, administration of the xanthine oxidase inhibitor in combination with the active oxygen scavenger conducted according to the present invention can suppress viral proliferation.

## Claims

1. An antiviral agent comprising a xanthine oxidase inhibitor in combination with an active oxygen scavenger.

2. The antiviral agent according to claim 1, wherein the xanthine oxidase inhibitor is one or more members selected from the group consisting of Allopurinol, Febuxostat, and Topiroxostat.

3. The antiviral agent according to claim 1 or 2,
wherein the active oxygen scavenger is one or more members selected from the group consisting of a reduced-form thiol agent, ascorbic acid or a derivative thereof, a tocopherol, an astaxanthine, and a flavonoid.

4. The antiviral agent according to any one of claims 1 to 3, which is an antiviral agent composition comprising a xanthine oxidase inhibitor and an active oxygen scavenger.

5. The antiviral agent according to any one of claims 1 to 3, which is a combination of a composition comprising a xanthine oxidase inhibitor with a composition comprising an active oxygen scavenger.

6. Use, for producing an antiviral agent, of a xanthine oxidase inhibitor in combination with an active oxygen scavenger.

7. A combination, for use in prevention or treatment of a viral infectious disease, of a xanthine oxidase inhibitor with an active oxygen scavenger.

8. A method for preventing or treating a viral infectious disease, comprising administering to a subject in need thereof a xanthine oxidase inhibitor in combination with an active oxygen scavenger.
